(19) Europäisches Patentamt
European Patent Office
Office européen des brevets

(11) Veröffentlichungsnummer : **0 058 868**
**B1**

(12) # EUROPÄISCHE PATENTSCHRIFT

(45) Veröffentlichungstag der Patentschrift : **11.04.84**

(51) Int. Cl.³ : **C 07 D239/10**, A 01 N 43/54

(21) Anmeldenummer : **82100905.7**

(22) Anmeldetag : **09.02.82**

(54) **Substituierte Tetrahydropyrimidinonderivate, Verfahren zu ihrer Herstellung und Herbizide, die diese Derivate als Wirkstoffe enthalten.**

(30) Priorität : **20.02.81 JP 23122/81**

(43) Veröffentlichungstag der Anmeldung : **01.09.82 Patentblatt 82/35**

(45) Bekanntmachung des Hinweises auf die Patenterteilung : **11.04.84 Patentblatt 84/15**

(84) Benannte Vertragsstaaten : **AT BE CH DE FR GB IT LI NL SE**

(56) Entgegenhaltungen : **DE-A- 2 555 582**

(73) Patentinhaber : **NIHON TOKUSHU NOYAKU SEIZO K.K.**
**No.4, 2-chome, Nihonbashi Honcho Chuo-ku**
**Tokyo 103 (JP)**

(72) Erfinder : **Aya, Masahiro**
**2-844, Suzuki-cho**
**Kodaira-shi Tokyo (JP)**
Erfinder : **Saito, Junichi**
**3-7-12, Osawa**
**Mitaka-shi Tokyo (JP)**
Erfinder : **Yasui, Kazuomi**
**7-6-15, Shakujii-dai**
**Nerima-ku Tokyo (JP)**
Erfinder : **Shiokawa, Kozo**
**210-6, Shukugawara Tama-ku**
**Kawasaki-shi Kanagawa-ken (JP)**

(74) Vertreter : **Gremm, Joachim, Dr. et al**
**Bayer AG c/o Zentralbereich Patente, Marken und Lizenzen**
**D-5090 Leverkusen 1, Bayerwerk (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 058 868 B1

Jouve, 18, rue St-Denis, 75001 Paris, France

Substituierte Tetrahydropyrimidinonderivate, Verfahren zu ihrer Herstellung und Herbizide, die diese Derivate als Wirkstoffe enthalten.

Die Erfindung betrifft substituierte Tetrahydropyrimidinonderivate, Verfahren zur Herstellung dieser Derivate und Herbizide, die diese Derivate als Wirkstoffe enthalten.

Die Erfindung betrifft insbesondere substituierte Tetrahydropyrimidinderivate, die die allgemeine Formel

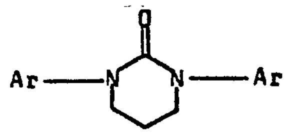

(I)

haben, worin die Gruppen Ar gleich oder verschieden sein können und jeweils aus einer α-Naphthylgruppe und einer Gruppe der Formel

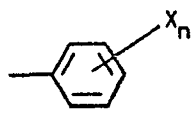

ausgewählt sind, worin X ein Rest ist, der aus einem Halogenatom, einem Alkylrest mit 1-4 C-Atomen, einem Alkoxyrest mit 1-4 C-Atomen, einer Nitrogruppe, einer Cyanogruppe, einer Alkylcarbonylgruppe mit 1-4 C-Atomen im Alkylteil, einer Alkoxycarbonylgruppe mit 1-4 C-Atomen im Alkoxyteil, einem Trifluormethylrest und einer Phenoxygruppe bestehenden Gruppe ausgewählt ist, wobei in Fällen, in denen mehrere Gruppen X vorhanden sind, diese gleich oder verschieden sein können, und n für 0, 1, 2 oder 3 steht.

Die substituierten Tetrahydropyrimidinonderivate der vorstehenden allgemeinen Formel (I) können beispielsweise nach den nachstehend beschriebenen Verfahren (A), (B) oder (C) hergestellt werden, so daß die Erfindung auch diese Herstellungsverfahren umfaßt.

(A) Ein Verfahren zur Herstellung eines substituierten Tetrahydropyrimidinonderivats der vorstehenden allgemeinen Formel (I), das gekennzeichnet ist durch Umsetzung einer Verbindung der allgemeinen Formel

$$Ar—NH(CH_2)_3OH \qquad (II)$$

in der Ar die vorstehend genannte Bedeutung hat, und eines Isocyanats der allgemeinen Formel

$$Ar—N=C=O \qquad (III)$$

in der Ar die vorstehend genannte Bedeutung hat, und anschließende Umsetzung mit einem Halogenierungsmittel und dann mit einem Alkalihydroxid.

(B) Ein Verfahren zur Herstellung eines substituierten Tetrahydropyrimidinonderivats der vorstehenden allgemeinen Formel (I), das gekennzeichnet ist durch Umsetzung einer Verbindung der allgemeinen Formel

$$Ar—NH(CH_2)_3Y \qquad (IV)$$

in der Ar die vorstehend genannte Bedeutung hat und Y ein Halogenatom ist, und eines Isocyanats der allgemeinen Formel

$$Ar—N=C=O \qquad (III)$$

in der Ar die vorstehend genannte Bedeutung hat, und anschließende Umsetzung mit einem Alkalihydroxid.

(C) Ein Verfahren zur Herstellung eines substituierten Tetrahydropyrimidinonderivats der vorstehenden allgemeinen Formel (I), das gekennzeichnet ist durch Umsetzung einer Verbindung der allgemeinen Formel

$$Ar—NH(CH_2)_3NH—Ar \qquad (V)$$

in der die Gruppen Ar die vorstehend genannte Bedeutung haben, mit Phosgen ($COCl_2$) oder Trichloromethylchloroformiat ($CCl_3OCOCl$).

Die Erfindung umfaßt ferner Herbizide, die die substituierten Tetrahydropyrimidinonderivate der vorstehenden allgemeinen Formel (I) als Wirkstoffe enthalten.

Von der Anmelderin wurden umfassende Untersuchungen mit dem Ziel durchgeführt, neue Verbindungen mit ausgezeichneter selektiver herbizider Aktivität herzustellen. Zunächst wurde dem Mechanismus dieser Wirkung der zur Zeit verfügbaren Verbindungen mit herbizider Aktivität, insbesondere dem der Harnstoffverbindungen Aufmerksamkeit gewidmet und in dieser Richtung geforscht. Als Ergebnis gelang der Anmelderin die Synthetisierung der substituierten Tetrahydropyrimidinonderivate der vorstehenden allgemeinen Formel (I). Ferner wurde nun gefunden, daß die Verbindungen der Formel (I) eine außergewöhnlich gute selektive herbizide Aktivität aufweisen und einen genauen herbiziden Effekt hervorbringen und insbesondere die Chlorose als Folge der Hemmung der Chlorophyllsynthese bewirken.

In Chemical Abstracts, Vol. 57, 9860a, 1962, wird eine Verbindung beschrieben, von der angenommen werden könnte, daß sie eine gewisse Ähnlichkeit in der chemischen Struktur mit den Verbindungen gemäß der Erfindung haben könnte. Es handelt sich um die Verbindung 1-Methyl-3-phenylhexahydro-2-pyrimidinon. Ihre Verwendung wird jedoch in diesem Artikel in keiner Weise erwähnt.

Die Untersuchungen der Anmelderin haben ergeben, daß, während die in der vorstehend genannten Veröffentlichung beschriebene bekannte Verbindung fast keine herbizide Wirkung aufweist, wie der später beschriebene Vergleichsversuch zeigt, die neuen Verbindungen der vorstehenden allgemeinen Formel (I) eine ganz ausgezeichnete selektive herbizide Aktivität zeigen.

Ferner wurde gefunden, daß von den Verbindungen der vorstehenden allgemeinen Formel (I) diejenigen, in denen der das Stickstoffatom in 1- oder 3-Stellung substituierende Phenylrest wenigstens einen Substituenten hat und dieser Substituent in m-Stellung steht, eine besonders ausgezeichnete Aktivität aufweisen.

Gegenstand der Erfindung sind demgemäß die Verbindungen der vorstehenden allgemeinen Formel (I), Verfahren zu ihrer Herstellung und ihre Verwendung als Herbizide.

Die vorstehend beschriebenen Ziele sowie verschiedene andere Ziele und Vorteile der Erfindung ergeben sich aus der folgenden Beschreibung.

Da die Verbindungen gemäß der Erfindung nur eine geringe Toxizität für Warmblüter und, wie vorstehend erwähnt, gute Selektivität aufweisen, können sie in zweckmäßiger und einfacher Weise für die Unkrautbekämpfung verwendet werden. Die Verbindungen gemäß der Erfindung weisen ferner eine überlegene selektive Bekämpfungswirkung auf, besonders wenn sie als Vorauflauf- oder Nachauflauf-Behandlungsmittel für Reisfeldunkräuter verwendet werden.

Die Sicherheit der Verbindungen gemäß der Erfindung ist ausgezeichnet, und sie weisen eine überlegene herbizide Aktivität und ein weites herbizides Spektrum auf.

Beispielsweise zeigen sie herbizide Aktivität gegen die nachstehend genannten Paddyfeld-Unkräuter, ohne irgendeine schädliche Wirkung auf die Paddyreispflanzen aufzuweisen.

Paddyfeld-Unkräuter
Dikotyledone
Rotala indica Koehne,
Lindernia Procumbens Philcox,
Ludwiga prostrata Roxburgh,
Potamogeton distinctus A. Benn,
Elatine triandra Schk usw.

Monokotyledone
Echinochloa crus-galli Beauv. var,
Monochoria vaginalis Presl,
Eleocharis acicularis L.,
Eleocharis Kuroguwai Ohwi,
Cyperus difformis L.,
Cyperus serotinus Rottboel,
Sagittaria pygmea Miq,
Alisma canaliculatum A. Br. et Bouche,
Scirpus juncoides Roxburgh var. usw.

Darüber hinaus zeigen die Verbindungen herbizide Aktivität gegen die nachstehend genannten Unkräuter, ohne irgendeine nachteilige Wirkung auf Nutzpflanzen einschließlich der Dikotyledone wie Senf, Senfpflanzen, Baumwolle, Möhren, Erbsen und Bohnen, Kartoffeln, Runkelrüben, Java-Mandeln usw. und Monokotyledone wie Mais, Reis, Hafer, Gerste, Weizen, Hirse, Zuckerrohr usw. auszuüben.

Feldunkräuter-Dikotyledone
Polygonum sp.,
Chenopodium album Linnaeus,
Stellaria media Villars,

Portulaca oleracea Linnaeus usw.
Monokotyledone
Echinochloa crus-galli Beauv. var.,
Digitaria adscendens Henr.,
Cyperus iria L. usw.

Es ist zu bemerken, daß die vorstehend genannten Arten von Pflanzen lediglich als repräsentativ für die in Lateinisch genannten jeweiligen Gattungen angegeben wurden.

Ferner ist die Anwendbarkeit der Wirkstoffe der Formel (I) gemäß der Erfindung nicht auf Paddyfeldunkräuter und Feldunkräuter begrenzt, vielmehr sind sie auch wirksam gegen Unkräuter wie Binsen oder Unkräuter auf Brachland u. dgl. Der hier gebrauchte Ausdruck « Unkräuter » bedeutet in seinem weitesten Sinne alle Unkräuter, die dort wachsen, wo ihr Wachstum unerwünscht ist.

Die substituierten Tetrahydropyrimidinonderivate der vorstehenden allgemeinen Formel (I) gemäß der Erfindung, können beispielsweise nach den folgenden Verfahren (A), (B) oder (C) hergestellt werden.

Verfahren (A)

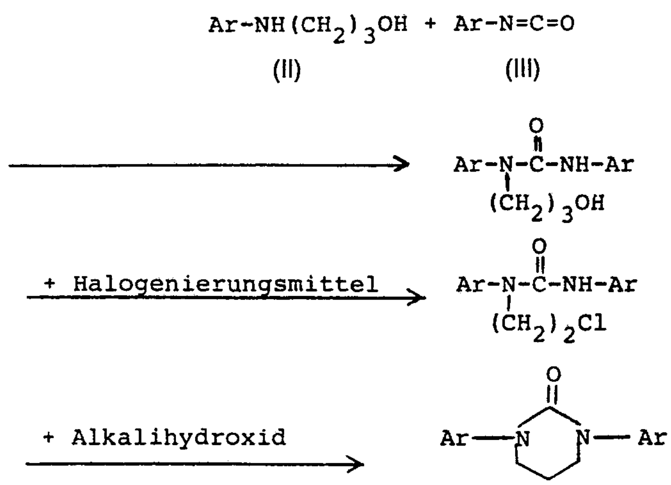

(II) (III)

(I)

Hierin haben die Gruppen Ar die vorstehend genannte Bedeutung.

Im vorstehenden Reaktionsschema steht jede Gruppe Ar für eine α-Naphthylgruppe oder eine Gruppe der Formel

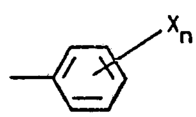

wobei als spezielle Beispiele für X Halogenatome wie Fluor, Chlor, Brom, Jod usw., niedere Alkylreste wie Methyl, Ethyl, n-Propyl oder Isopropyl, n-Butyl, Isobutyl, sek.- oder t-Butyl usw., niedere Alkoxyreste mit ähnlichen Alkylresten, wie sie vorstehend genannt wurden, eine Nitrogruppe, eine Cyanogruppe, niedere Alkylcarbonylgruppen mit ähnlichen Alkylresten, wie sie vorstehend genannt wurden, niedere Alkoxycarbonylgruppen mit ähnlichen Alkoxyresten wie sie vorstehend genannt wurden, eine Trimethylfluoromethylgruppe und eine Phenoxygruppe zu nennen sind, während n für 0, 1, 2 oder 3 steht. Wenn hier mehrere Gruppen X vorhanden sind, können sie gleich oder verschieden sein.

Für das durch das vorstehende Reaktionsschema (A) veranschaulichte Verfahren zur Herstellung der Verbindungen gemäß der Erfindung seien als spezielle Beispiele eines der Ausgangsmaterialien, nämlich der Verbindung der allgemeinen Formel (II), genannt: N-3-Hydroxypropyl-3-fluoroanilin, N-3-Hydroxypropyl-3-chloroanilin, N-3-Hydroxypropyl-3-bromoanilin, N-3-Hydroxypropyl-3-toluidin, N-3-Hydroxypropyl-3-methoxyanilin, N-3-Hydroxypropyl-3-isopropoxyanilin, N-3-Hydroxypropyl-3-phenoxyanilin, N-3-Hydroxypropyl-3-nitroanilin, N-3-Hydroxypropyl-3-cyanoanilin, N-3-Hydroxypropyl-3-acetanilin, N-3-Hydroxypropyl-3-ethoxycarbonylanilin, N-3-Hydroxypropyl-3-trifluoromethylanilin, N-3-Hydroxypropyl-2,5-dichloroanilin, N-3-Hydroxypropyl-3,4-dichloroanilin, N-3-Hydroxypropyl-3,5-dichloroanilin, N-3-Hydroxypropyl-3,5-xylidin, N-3-Hydroxypropyl-3,4-dimethoxyanilin, N-3-Hydroxypropyl-3-chlor-4-trifluoromethylanilin, N-3-Hydroxypropyl-3,5-bistrifluoromethylanilin, N-3-Hydroxypropyl-2,4,5-trichloranilin, N-3-Hydroxypropyl-3,5-dimethoxyanilin, N-3-Hydroxypropylanilin, N-3-Hydroxypropyl-α-naphthylamin, N-3-Hydroxypropyl-2-chloroanilin, N-3-Hydroxypropyl-4-chloroanilin, N-3-Hydroxypropyl-2-toluidin, N-3-Hydroxypropyl-4-toluidin, N-3-Hydroxypropyl-2-methoxyanilin, N-3-Hydroxypropyl-4-methoxyanilin, N-3-Hydroxypropyl-2-trifluoromethylanilin, N-3-Hydroxypropyl-4-trifluoromethylanilin, N-3-Hydroxypropyl-4-isopropylanilin, N-3-Hydroxypropyl-2,6-xylidin und N-3-Hydroxypropyl-3,4-xylidin.

Spezielle Beispiele des Isocyanats der allgemeinen Formel (III), des anderen Ausgangsmaterials, sind Phenylisocyanat, α-Naphthylisocyanat, 2-Chlorphenylisocyanat, 3-Chlorophenylisocyanat, 4-Chlorophenylisocyanat, 2-Tolylisocyanat, 3-Tolylisocyanat, 4-Tolylisocyanat, 2-Methoxyphenylisocyanat, 3-Methoxyphenylisocyanat, 4-Methoxyphenylisocyanat, 3-Nitrophenylisocyanat, 2-Trifluoromethylphenylisocyanat, 3-Trifluoromethylphenylisocyanat, 4-Trifluoromethylphenylisocyanat, 4-Isopropylphenylisocyanat, 3,5-Dichlorophenylisocyanat, 2,6-Xylylisocyanat, 3,4-Xylylisocyanat, 3-Chloro-4-trifluoromethylphenylisocyanat, 2,4,5-Trichlorophenylisocyanat, 3-Fluorophenylisocyanat, 3-Bromophenylisocyanat, 3-Isopropoxyphenylisocyanat, 3-Phenoxyphenylisocyanat, 3-Cyanophenylisocyanat, 3-Acetylphenylisocyanat, 3-Ethoxycarbonylphenylisocyanat, 2,5-Dichlorophenylisocyanat, 3,4-Dichlorophenylisocyanat, 3,5-Xylylisocyanat, 3,4-Dimethoxyphenylisocyanat, 3,5-Bistrifluoromethylisocyanat, 3,5-Dimethoxyphenylisocyanat usw.

Auch bei dem vorstehend beschriebenen Herstellungsverfahren (A) kommen als Halogenierungsmittel, die bei der Reaktion zu verwenden sind, beispielsweise Thionylchlorid, Phosphortrichlorid, Phosphortribromid und Salzsäure in Frage.

Ferner kommen bei dem vorstehend beschriebenen Herstellungsverfahren (A) als Alkalihydroxide, die bei der Reaktion zu verwenden sind, beispielsweise Natriumhydroxid, Kaliumhydroxid und Lithiumhydroxid in Frage.

Das vorstehend beschriebene Herstellungsverfahren wird durch das folgende repräsentative Beispiel ausführlicher beschrieben :

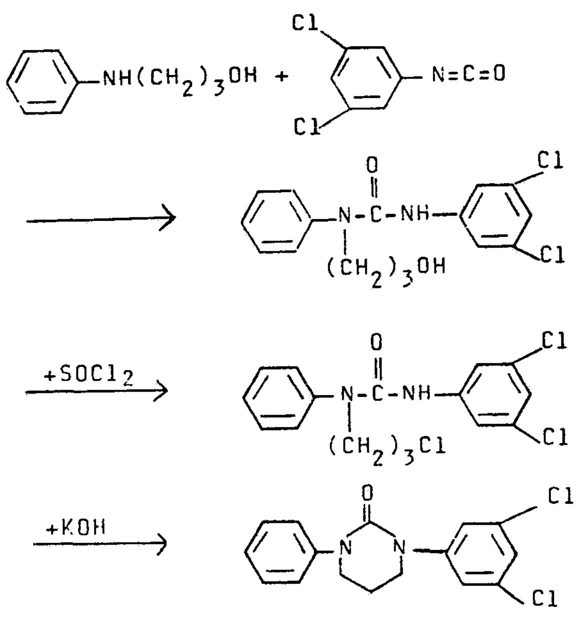


Dieses Verfahren zur Herstellung der Verbindungen gemäß der Erfindung wird zweckmäßig unter Verwendung eines Lösungsmittels oder eines Verdünnungsmittels durchgeführt. Zu diesem Zweck können beliebige inerte Lösungsmittel oder Verdünnungsmittel verwendet werden.

Beispiele solcher Lösungsmittel und Verdünnungsmittel sind Wasser, aliphatische, alicyclische und aromatische Kohlenwasserstoffe (die gegebenenfalls chloriert sein können), z. B. Hexan, Cyclohexan, Petroläther, Ligroin, Benzol, Toluol, Xylol, Methylenchlorid, Chloroform, Tetrachlorkohlenstoff, Ethylenchlorid, Trichloroethylen und Chlorbenzol, Äther, z. B. Diethyläther, Methylethyläther, Diisopropyläther, Dibutyläther, Propylenoxid, Dioxan und Tetrahydrofuran, Ketone, z. B. Aceton, Methylethylketon, Methylisopropylketon und Methylisobutylketon, Nitrile, z. B. Acetonitril, Propionitril und Acrylnitril, Alkohole, z. B. Methanol, Ethanol, Isopropanol, Butanol und Ethylenglykol, Ester, z. B. Ethylacetat und Amylacetat, Säureamide, z. B. Dimethylformamid und Dimethylacetamid, Sulfone und Sulfoxide, z. B. Dimethylsulfoxid und Sulfolan, und Basen, z. B. Pyridin.

Ferner kann die Reaktion beim Herstellungsverfahren gemäß der Erfindung kontinuierlich ohne Abtrennung der Zwischenprodukte während des Reaktionsprozesses durchgeführt werden, und sie vermag eine gewünschte Verbindung gemäß der Erfindung von hoher Reinheit in hoher Ausbeute zu liefern.

Das Verfahren gemäß der Erfindung kann innerhalb eines weiten Bereichs von Temperaturen durchgeführt werden. Im allgemeinen wird es bei einer Temperatur zwischen − 20 °C und dem Siedepunkt des Gemisches, vorzugsweise zwischen 0 °C und 100 °C durchgeführt. Die Reaktion wird

vorzugsweise unter Normaldruck durchgeführt, kann jedoch auch unter erhöhtem oder vermindertem Druck durchgeführt werden.

Verfahren (B)

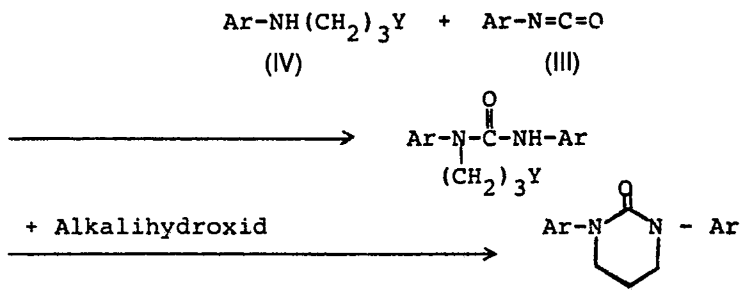


(IV) (III)

(I)

Hierin haben die Gruppen Ar und Y die vorstehend genannten Bedeutungen.

Für das durch das vorstehende Reaktionsschema veranschaulichte Verfahren zur Herstellung der Verbindungen gemäß der Erfindung seien als spezielle Beispiele des einen Ausgangsmaterials, nämlich der Verbindung der allgemeinen Formel (IV), genannt : N-3-Chloropropyl-3-fluoroanilin, N-3-Chloropropyl-3-chloroanilin, N-3-Chloropropyl-3-bromoanilin, N-3-Chloropropyl-3-toluidin, N-3-Chloropropyl-3-methoxyanilin, N-3-Chloropropyl-3-isopropoxyanilin, N-3-Chloropropyl-3-phenoxyanilin, N-3-Chloropropyl-3-nitroanilin, N-3-Chloropropyl-3-cyanoanilin, N-3-Chloropropyl-3-acetylanilin, N-3-Chloropropyl-3-ethoxycarbonylanilin, N-3-Chloropropyl-3-trifluoromethylanilin, N-N-3-Chloropropyl-2,5-dichloroanilin, N-3-Chloropropyl-3,4-dichloroanilin, N-3-Chloropropyl-3,5-dichloroanilin, N-3-Chloropropyl-3,5-xylidin, N-3-Chloropropyl-3,4-dimethoxyanilin, N-3-Chloropropyl-3-chloro-4-trifluoromethylanilin, N-3-Chloropropyl-3,5-bistrifluoromethylanilin, N-3-Chloropropyl-2,4,5-trichloroanilin, N-3-Chloropropyl-3,5-dimethoxyanilin, N-3-Chloropropylanilin, N-3-Chloropropyl-α-naphthylanilin, N-3-Chloropropyl-2-chloroanilin, N-3-Chloropropyl-4-chloroanilin, N-3-Chloropropyl-2-toluidin, N-3-Chloropropyl-4-toluidin, N-3-Chloropropyl-2-methoxyanilin, N-3-Chloropropyl-4-methoxyanilin, N-3-Chloropropyl-2-trifluoromethylanilin, N-3-Chloropropyl-4-trifluoromethylanilin, N-3-Chloropropyl-4-isopropylanilin, N-3-Chloropropyl-2,6-xylidin, N-3-Chloropropyl-3,4-xylidin usw. sowie ihre analogen N-3-Brompropylverbindungen.

Als spezielle Beispiele des Isocyanats der allgemeinen Formel (III), des anderen Ausgangsmaterials, können die gleichen Verbindungen, die für das vorstehend beschriebene Verfahren (I) angegeben wurden, genannt werden.

Ebenso können als spezielle Beispiele des bei der Reaktion des vorstehenden Verfahrens (B) zu verwendenden Alkalihydroxids die gleichen Verbindungen, die für das Verfahren (A) angegeben wurden, genannt werden.

Das vorstehend beschriebene Herstellungsverfahren (B) wird durch das folgende repräsentative Beispiel ausführlicher beschrieben :

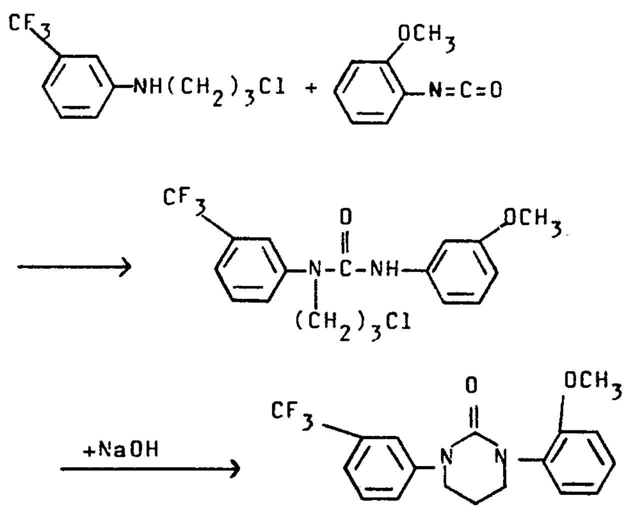


Zur Durchführung des vorstehend dargestellten Verfahrens wird zweckmäßig ein inertes Lösungsmittel oder Verdünnungsmittel ähnlich den vorstehend beschriebenen verwendet, wodurch die gewünschte Verbindung in hoher Reinheit und in hoher Ausbeute erhalten werden kann. Die Reaktion kann

kontinuierlich ohne Abtrennung der Zwischenprodukte während des Reaktionsprozesses durchgeführt werden.

Das Verfahren (B) kann auch wirksam in Gegenwart eines Katalysators durchgeführt werden. Beispiele geeigneter Katalysatoren sind Tetrabutylammoniumbromid u. dgl.

Das Verfahren (B) kann ebenfalls in einem weiten Bereich von Temperaturen durchgeführt werden. Im allgemeinen wird es bei einer Temperatur zwischen − 20 °C und dem Siedepunkt des Gemisches, vorzugsweise zwischen 0 °C und 100 °C durchgeführt. Die Reaktion wird vorzugsweise unter Normaldruck durchgeführt, jedoch kann auch unter erhöhtem oder vermindertem Druck gearbeitet werden.

Verfahren (C)

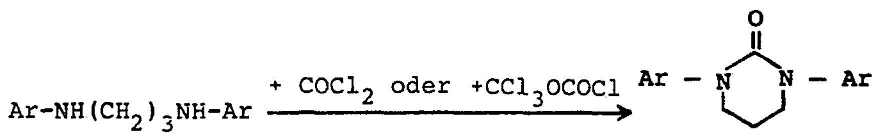


(I)

Hierin haben die Gruppen Ar die vorstehend genannte Bedeutung.

Für das durch das vorstehende Reaktionsschema (C) veranschaulichte Verfahren zur Herstellung der Verbindungen gemäß der Erfindung seien als spezielle Beispiele der Ausgangsverbindung der allgemeinen Formel (V) genannt : 1-(3-Fluoroanilino)-3-anilinopropan, 1-(3-Chloroanilino)-3-anilinopropan, 1-(3-Bromoanilino)-3-anilinopropan, 1-(3-Toluidino)-3-anilinopropan, 1-(3-Methoxyanilino)-3-anilinopropan, 1-(3-Isopropoxyanilino)-3-anilinopropan, 1-(3-Phenoxyanilino)-3-anilinopropan, 1-(3-Nitroanilino)-3-anilinopropan, 1-(3-Cyanoanilino)-3-anilinopropan, 1-(3-Acetylanilino)-3-anilinopropan, 1-(3-Ethoxycarbonylanilino)-3-anilinopropan, 1-(3-Trifluoromethylanilino)-3-anilinopropan, 1-(3-Trifluoromethylanilino)-3-α-naphthylaminopropan, 1-(3-Fluoroanilino)-3-(3-toluidino)propan, 1-(3-Chloroanilino)-3-(2-chloroanilino)propan, 1-(3-Chloroanilino)-3-(3-chloroanilino)propan, 1-(3-Chloroanilino)-3-(4-chloroanilino)-propan, 1-(3-Chloroanilino)-3-(2-toluidino)propan, 1-(3-Chloroanilino)-3-(3-toluidino)-propan, 1-(3-Chloroanilino)-3-(4-toluidino)propan, 1-(3-Chloroanilino)-3-(2-methoxyanilino)propan, 1-(3-Chloroanilino)-3-(3-methoxyanilino)propan, 1-(3-Chloroanilino)-3-(4-methoxyanilino)propan, 1-(3-Chloroanilino)-3-(3-nitroanilino)propan, 1-(3-Chloroanilino)-3-(2-trifluoromethylanilino)propan, 1-(3-Chloroanilino)-3-(3-trifluoromethylanilino)propan, 1-(3-Chloroanilino)-3-(4-trifluoromethylanilino)propan, 1-(3-Bromoanilino)-3-(3-trifluoromethylanilino)propan, 1-(3-Toluidino)-3-(2-toluidino)propan, 1,3-Bis(3-toluidino)propan, 1-(3-Toluidino)-3-(4-toluidino)-propan, 1,3-Bis(3-methoxyanilino)propan, 1-(3-Methoxyanilino)-3-(3-trifluoromethylanilino)propan, 1-(3-Nitroanilino)-3-(2-toluidino)propan, 1-(3-Cyanoanilino)-3-(3-trifluoromethylanilino)propan, 1-(3-Trifluoromethylanilino)-3-(2-chloroanilino)propan, 1-(3-Trifluoromethylanilino)-3-(4-chloroanilino)propan, 1-(3-Trifluoromethylanilino)-3-(2-toluidino)propan, 1-(3-Trifluoromethylanilino)-3-(3-toluidino)propan, 1-(3-Trifluoromethylanilino)-3-(4-isopropylanilino)propan, 1-(3-Trifluoromethylanilino)-3-(2-methoxyanilino)propan, 1-(3-Trifluoromethylanilino)-3-(4-methoxyanilino)propan, 1,3-Bis(3-trifluoromethylanilino)propan, 1-(2,5-Dichloroanilino)-3-anilinopropan, 1-(3,4-Dichloroanilino)-3-anilinopropan, 1-(3,5-Dichloroanilino)-3-anilinopropan, 1-(3,5-Xylidino)-3-anilinopropan, 1-(3,4-Dimethoxyanilino)-3-anilinopropan, 1-(3-Chloro-4-trifluoromethylanilino)-3-anilinopropan, 1-(3,5-Bistrifluoromethylanilino)-3-anilinopropan, 1-(3-Chloroanilino)-3-(3,5-dichloroanilino)propan, 1-(3-Trifluoromethylanilino)-3-(3,5-dichloroanilinopropan, 1-(3-Trifluoromethylanilino)-3-(2,6-xylidino)propan, 1-(3-Trifluoromethylanilino)-3-(3,4-xylidino)propan, 1-(3-Trifluoromethylanilino)-3-(3-chloro-4-trifluoromethylanilino)propan, 1-(3,4-Dichloroanilino)-3-(2-toluidino)propan, 1-(2,4,5-Trichloroanilino)-3-anilinopropan, 1-(3-Trifluoromethylanilino)-3-(2,4,5-trichloroanilino)propan, 1-(3,5-Dimethoxyanilino)-3-anilinopropan, 1-(3-Phenoxyanilino)-3-(3-trifluoromethylanilino)propan, 1-(3,5-Dichloroanilino)-3-(2-methoxyanilino)propan und 1-α-Naphthylamino-3-anilinopropan.

Das vorstehend beschriebene Herstellungsverfahren (C) wird durch das folgende repräsentative Beispiel ausführlicher beschrieben :

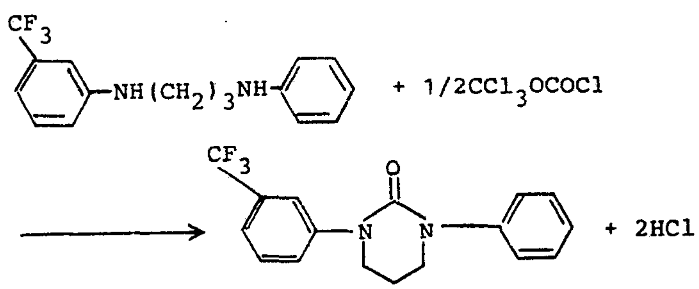


Zur Durchführung des vorstehend dargestellten Verfahrens (C) wird zweckmäßig ein inertes Lösungsmittel oder Verdünnungsmittel ähnlich den vorstehend beschriebenen verwendet, wodurch die

gewünschte Verbindung in hoher Reinheit und in hoher Ausbeute erhalten werden kann. Die Reaktion kann in Gegenwart eines säurebindenden Mittels durchgeführt werden. Als säurebindende Mittel eignen sich beispielsweise die üblicherweise verwendeten Mittel, z. B. Alkalihydroxide, -carbonate, -bicarbonate und -alkoholate, und tertiäre Amine, z. B. Triethylamin, Diethylanilin und Pyridin.

Zur Herstellung der aktiven Verbindungen gemäß der Erfindung unter Anwendung des Verfahrens (C) kann die Reaktion innerhalb eines weiten Bereichs von Temperaturen durchgeführt werden. Im allgemeinen kann sie bei einer Temperatur zwischen – 20 °C und dem Siedepunkt des Gemisches, zweckmäßig zwischen 0 °C und 100 °C durchgeführt werden. Ferner wird die Reaktion zweckmäßig unter Normaldruck durchgeführt, jedoch kann auch bei erhöhtem oder vermindertem Druck gearbeitet werden.

Für die Verwendung als Herbizide können die Verbindungen gemäß der Erfindung direkt nach Verdünnung mit Wasser angewandt werden, oder sie können zu verschiedenen Präparaten nach üblicherweise auf dem Gebiet der Herstellung landwirtschaftlicher Chemikalien angewandten Methoden unter Verwendung landwirtschaftlicher Hilfsstoffe konfektioniert und angewandt werden. Diese verschiedenen Präparate können zur praktischen Anwendung als solche oder nach Verdünnung mit Wasser auf eine gewünschte Konzentration verwendet werden. Der hier gebrauchte Ausdruck « landwirtschaftliche Hilfsstoffe » bedeutet beispielsweise Verdünnungs- und Streckmittel (Lösungsmittel, Füllstoffe, Träger usw.), oberflächenaktive Mittel (Lösungsvermittler, Emulgatoren, Dispergiermittel, Netzmittel usw.), Stabilisatoren, Haftmittel, Treibmittel für Aerosole, Synergisten usw.

Beispiele geeigneter Lösungsmittel sind Wasser und organische Lösungsmittel, z. B. Kohlenwasserstoffe (z. B. n-Hexan, Petroläther, Naphtha, Erdölfraktionen (z. B. Paraffine, Kerosin, Leichtöl, Mittelöl und Schweröl), Benzol, Toluol und Xylol), halogenierte Kohlenwasserstoffe (z. B. Methylenchlorid, Tetrachlorkohlenstoff, Trichlorethylen, Ethylenchlorid, Ethylendibromid, Chlorbenzol, Chloroform usw.), Alkohole (z. B. Methylalkohol, Ethylalkolol, Propylalkohol und Ethylenglykol), Äther (z. B. Diethyläther, Ethylenoxid und Dioxan), Alkoholäther (z. B. Ethylenglykolmonomethyläther), Ketone (z. B. Aceton und Isophoron), Ester (z. B. Ethylacetat und Amylacetat), Amide (z. B. Dimethylformamid und Dimethylacetamid), Sulfoxide (z. B. Dimethylsufoxid).

Beispiele geeigneter Füllstoffe oder Träger sind anorganische pulverförmige Feststoffe, z. B. Schwefel, gelöschter Kalk, Magnesiumkalk, Gips, Calciumcarbonat, Siliciumdioxid, Perlit, Bimsstein, Kalzit, Diatomit, amorphes Siliciumdioxid, Tonerde, Zeolithe, Tonminerale (z. B. Pyrophyllit, Talkum, Montmorillonit, Beidellit, Vermiculit, Kaolinit und Glimmer), pflanzliche pulverförmige Feststoffe, z. B. Getreidemehl, Stärke, verarbeitete Stärken, Zucker, Glucose und Produkte aus zerkleinerten Pflanzenstengeln und gemahlene feste synthetische Harze, z. B. Phenolharze, Harnstoffharze und Vinylchloridharze.

Beispiele geeigneter oberflächenaktiver Mittel sind anionaktive Tenside, z. B. Alkylsulfate (z. B. Natriumlaurylsulfat), Arylsulfonsäuren (z. B. Alkylarylsulfonat und Natrium-Alkylnaphthalinsulfonat), Bernsteinsäuresalze und Schwefelsäureestersalze von Polyethylen glykol-alkylaryläthern, kationaktive Tenside, z. B. Alkylamine (z. B. Laurylamin, Stearyltrimethylammoniumchlorid und Alkyldimethylbenzylammoniumchlorid) und Polyoxyethylenalkylamine, nichtionogene Tenside, z. B. Polyoxyethylenglykolether (z. B. Polyoxyethylenalkylarylether und seine Kondensate), Polyoxyethylenglykolester (z. B. Polyoxyethylenfettsäureester), Polyolester (z. B. Polyoxyethylensorbitanmonolaurat), ampholytische oberflächenaktive Mittel usw.

Beispiele weiterer Hilfsstoffe sind Stabilisatoren, Haftmittel (z. B. landwirtschaftliche Seifen, Kaseinleim, Natriumalginat, Polyvinylalkohol (PVA), Klebstoffe auf Vinylacetatbasis, Acrylklebstoffe usw.), Dispersionsstabilisatoren (z. B. Kasein, Tragant, Carboxymethylcellulose (CMC) und Polyvinylalkohol (PVA), und Synergisten).

Die Verbindungen gemäß der Erfindung können nach Verfahren, die üblicherweise auf dem Gebiet der Herstellung landwirtschaftlicher Chemikalien angewandt werden, zu verschiedenen Präparaten verarbeitet werden. Als Präparate kommen Emulsionen, Öllösungen, netzbare Pulver, Lösungen, Suspensionen, Pulver, Granulat, pulverförmige Feststoffe, Kapseln usw. in Frage.

Die Herbizide gemäßt der Erfindung können die vorstehend genannten Wirkstoffe in einer Menge von 0,001 bis 100 Gew.-%, vorzugsweise 0,005 bis 95 Gew.-%, enthalten.

Für den tatsächlichen Gebrauch kann der Gehalt an Wirkverbindungen in den vorstehend genannten Präparaten und gebrauchsfertigen Präparaten zweckmäßig im Bereich von 0,01 bis 95 Gew.-%, vorzugsweise 0,05 bis 60 Gew.-% liegen. Der Gehalt and diesen Wirkverbindungen kann in Abhängigkeit von der Form des Präparats, dem Verfahren, Zweck, der Zeit und dem Ort der Anwendung und der Schwere des Unkrautbefalls variiert werden.

Die Verbindungen gemäß der Erfindung können, falls weiter erforderlich, in Kombination mit anderen landwirtschaftlichen Chemikalien, z. B. Insektiziden, Fungiziden, Akariziden, Nematoziden, Antivirusmitteln, Herbiziden, Pflanzenwachstumsreglern, Lockmitteln (z. B. organischen Phosphatverbindungen, Carbamatverbindungen, Dithio- oder Thiocarbamatverbindungen, organischen Chlorverbindungen, Dinitroverbindungen, organischen Schwefel- oder Metallverbindungen, Antibiotika, substituierten Diphenylätherverbindungen, Harnstoffverbindungen und Triazinverbindungen, und/oder Düngemitteln u. dgl. verwendet werden.

Die verschiedenen Präparate und gebrauchsfertigen Präparate, die die vorstehend genannten

aktiven Verbindungen gemäß der Erfindung enthalten, können nach den für landwirtschaftliche Chemikalien üblichen Methoden angewandt werden, beispielsweise durch Sprühen, Vernebeln, Zerstäuben, Stäuben, Streuen von Granulat, Aufbringen auf Wasseroberflächen, Gießen usw.), Anwendung auf den Boden (z. B. Einarbeiten in den Boden und Berieseln) u. dgl. Ferner können sie nach dem ULV-Verfahren (ultra-low-volume method) angewandt werden. Bei diesem Verfahren kann das Präparat, sogar bis zu 100 % der aktiven Verbindungen enthalten.

Die Dosierung oder Aufwandmenge der Wirksubstanz pro Flächeneinheit beträgt etwa 0,1 bis 5 kg, vorzugsweise 0,2 bis 4 kg/ha. In besonderen Fällen ist es jedoch möglich oder manchmal sogar notwendig, eine über oder unter diesem Bereich liegende Aufwandmenge zu verwenden.

Die Erfindung umfaßt ein herbizides Mittel, das eine Verbindung der vorstehenden allgemeinen Formel (I) und ein Streckmittel (ein Lösungsmittel und/oder einen Füllstoff und/oder einen Träger) und/oder ein oberflächenaktives Mittel und, falls erforderlich, einen Stabilisator, ein Haftmittel und einen Synergisten enthält.

Die Erfindung umfaßt ferner ein Verfahren zur Unkrautbekämpfung, das dadurch gekennzeichnet ist, daß man auf die Unkräuter oder ihren Lebensraum eine Verbindung der vorstehenden allgemeinen Formel (I) allein oder in Kombination mit einem Streckmittel (einem Lösungsmittel und/oder Füllstoff und/oder Träger) und/oder einem oberflächenaktiven Mittel und, falls erforderlich, beispielsweise einem Stabilisator, einem Haftmittel und einem Synergisten aufbringt.

Die Ausführungsform der Erfindung wird nachstehend ausführlich durch die folgenden Beispiele beschrieben.

Beispiel 1 (netzbares Pulver)

15 Teile der Verbindung Nr. 6 gemäß der Erfindung, 80 Teile eines 1 : 5-Gemisches von gemahlenem Diatomit und gemahlenem Ton, 2 Teile Natriumalkylbenzolsulfonat und 3 Teile eines Kondensats von Natriumalkylnaphthalinsulfonat und Formalin wurden gemahlen und gemischt, wobei ein netzbares Pulver erhalten wurde. Dieses Pulver wurde mit Wasser verdünnt und durch Sprühen auf Unkräuter und/oder ihren Lebensraum angewandt.

Beispiel 2 (Emulsion)

30 Teile der Verbindung Nr. 10 gemäß der Erfindung, 55 Teile Xylol, 8 Teile Polyoxyethylenalkylphenylether und 7 Teile Calciumalkylbenzolsulfonat wurden unter Rühren gemischt, wobei eine Emulsion entstand. Diese Emulsion wurde mit Wasser verdünnt und durch Tropfen auf Unkräuter und/oder ihren Lebensraum angewandt.

Beispiel 3 (Stäubemittel)

2 Teile der Verbindung Nr. 25 gemäß der Erfindung und 98 Teile gemahlener Ton wurden gemahlen und gemischt, wobei ein Stäubemittel entstand. Dieses Mittel wurde auf Unkräuter und/oder ihren Lebensraum gestäubt.

Beispiel 4 (Stäubemittel)

1,5 Teile der Verbindung Nr. 40 gemäß der Erfindung, 0,5 Teile Isopropylhydrogenphosphat (PAP) und 98 Teile gemahlener Ton wurden gemahlen und gemischt, wobei ein Stäubemittel entstand, das auf Unkräuter und/oder ihren Lebensraum gestäubt wurde.

Beispiel 5 (Granulat)

20 Teile Wasser wurden zu einem Gemisch von 10 Teilen der Verbindung Nr. 1 gemäß der Erfindung, 30 Teilen Bentonit (Montmorillonit), 58 Teilen Talkum und 2 Teilen Ligninsulfonat gegeben. Das erhaltene Gemisch wurde innig geknetet, mit einem Extruder-Granulator zu Granulat einer Korngröße von 0,42 bis 2 mm (10-40 mesh) granuliert und bei 40 bis 50 °C getrocknet. Das Granulat wurde auf Unkräuter und/oder ihren Lebensraum gestreut.

Beispiel 6 (Granulat)

95 Teile eines Tonmineralgranulats einer Teilchengrößenverteilung von 0,2 bis 2 mm wurden in einen Drehmischer gegeben. Eine Lösung von 5 Teilen der Verbindung Nr. 2 gemäß der Erfindung in einem organischen Lösungsmittel wurde während des Drehens über die Teilchen gesprüht, wodurch diese gleichmäßig benetzt wurden. Sie wurden dann bei 40 bis 50 °C unter Bildung von Granulat getrocknet. Das Granulat wurde durch Streuen auf Unkräuter oder ihren Lebensraum angewandt.

Die neuen Verbindungen gemäß der Erfindung zeichnen sich im Vergleich zu den aus der Literatur bekannten aktiven Verbindungen mit ähnlichen Strukturen dadurch aus, daß sie eine wesentlich

verbesserte Wirkung und eine sehr geringe Toxizität gegenüber Warmblütern haben, so daß sie von großem Nutzen und Vorteil sind.

Die jede Erwartung übertreffenden ausgezeichneten Eigenschaften und die überaus starke Wirkung der Aktiven Verbindungen gemäß der Erfindung sind an den Ergebnissen der nachstehend beschriebenen Versuche erkennbar, in denen sie auf verschiedenen Unkräuter angewandt wurden.

Versuchsbeispiel 1

Test zur Behandlung von Stengeln, Blättern und des Bodens gegen Paddyfeld-Unkräuter unter wasserüberfluteten Bedingungen (Topfversuch)

Herstellung der aktiven Verbindung

Träger : 5 Gew.-Teile Aceton
Emulgator : 1 Gew.-Teil Benzyloxypolyglycolether

Ein die aktive Verbindung enthaltendes Präparat kann als Emulsion durch Mischen von 1 Gew.-Teil der aktiven Verbindung mit dem Aceton und Emulgator in den vorstehend genannten Mengen erhalten werden. Die vorbestimmte Dosis dieses Präparats wird für die tatsächliche Anwendung mit Wasser verdünnt.

Prüfmethode

Paddyfeldboden wurde in Wagner-Töpfe von 1/5 000 Ar gefüllt, und Paddyreissämlinge (Handelssorte « Kinmaze») in der 2-3-Blattphase (Höhe etwa 10 cm) wurden mit 2 Sämlingen pro Topf umgepflanzt. Ferner wurden Samen von Echinochloa crus-galli Beauv, var, Cyperus iria L, Monochoria vaginalis Presl, Scirpus juncoides Roxburgh var. und Breitblattunkräuter, Fragmente von Eleocharis acicularis L und Knollen von Cyperus serotinus Rottboel und Sagittaria pygmaea Miq geimpft, und jeder Topf wurde wassergefüllt gehalten. Wenn die Echinochloa curs-galli Beauv. var bis ungefähr zur 2-Blatt-Phase gewachsen war (etwa 7 bis 9 Tage nach dem Impfen), wurde der Wasserstand auf etwa 6 cm gebracht, und die vorbestimmte Menge jeder Testverbindung gemäß der Erfindung in Form einer Emulsion wurde unter Verwendung einer Pipette für die Behandlung angewandt. Nach dieser Behandlung wurde das Wasser um 2 bis 3 cm pro Tag für zwei Tage abgezogen, worauf die Wasserhöhe bei etwa 3 cm gehalten wurde, und am Tage vier Wochen nach der chemischen Behandlung wurden die herbizide Wirkung und das Ausmaß der Phytotoxizität bewertet und in 5 Bewertungen von 0 bis 5 nach den folgenden Maßstäben eingestuft :

Bewertung der Wirkung, ausgedrückt als Vernichtungsrate im Vergleich zu dem Fall der unbehandelten Fläche :

5 : 95 % oder mehr (vollständige Vernichtung)
4 : 80 % (einschließlich) bis 95 % (ausschließlich)
3 : 50 % (einschließlich) bis 80 % (ausschließlich)
2 : 30 % (einschließlich) bis 50 % (ausschließlich)
1 : 10 % (einschließlich) bis 30 % (ausschließlich)
0 : weniger als 10 % (keine Wirkung)

Bewertung der Phytotoxizität gegen Paddyreispflanzen, ausgedrückt als prozentuale Phytotoxizität im Vergleich zu dem Fall der unbehandelten Fläche :

5 : 90 % oder höher (tödlicher Schaden)
4 : 50 % (einschließlich) bis 90 % (ausschließlich)
3 : 30 % (einschließlich) bis 90 % (ausschließlich)
2 : 10 % (einschließlich) bis 30 % (ausschließlich)
1 : über 0 % und weniger als 10 %
0 : 0 % (keine Phytotoxizität)

Die Ergebnisse des Versuchs sind in Tabelle 1 genannt.

(Siehe Tabelle 1, Seite 11 ff.)

Tabelle 1

| Verbindung Nr. | Menge des Wirkstoffs, kg/ha | Herbizide Wirkung | | | | | | | | Phytotoxizität |
|---|---|---|---|---|---|---|---|---|---|---|
| | | Unkräuter | | | | | | | | |
| | | A | B | C | D | E | F | G | H | Reis |
| 1 | 2,0 | 5 | 5 | 5 | 5 | 5 | 5 | 5 | 5 | 0 |
| 2 | 2,0 | 5 | 5 | 5 | 5 | 5 | 5 | 5 | 5 | 0 |
| 3 | 2,0 | 5 | 5 | 5 | 5 | 5 | 5 | 5 | 5 | 0 |
| 4 | 2,0 | 5 | 5 | 5 | 5 | 5 | 5 | 5 | 5 | 0 |
| 5 | 2,0 | 5 | 5 | 5 | 5 | 5 | 5 | 5 | 5 | 0 |
| 6 | 2,0 | 5 | 5 | 5 | 5 | 5 | 5 | 5 | 5 | 0 |
| 7 | 2,0 | 5 | 5 | 5 | 5 | 5 | 5 | 5 | 5 | 0 |
| 8 | 2,0 | 5 | 5 | 5 | 5 | 5 | 5 | 5 | 5 | 0 |
| 9 | 2,0 | 5 | 5 | 5 | 5 | 5 | 5 | 5 | 5 | 0 |
| 10 | 2,0 | 5 | 5 | 5 | 5 | 5 | 5 | 5 | 5 | 0 |
| 12 | 2,0 | 5 | 5 | 5 | 5 | 5 | 5 | 5 | 5 | 0 |
| 15 | 2,0 | 5 | 5 | 5 | 5 | 5 | 5 | 5 | 5 | 0 |
| 16 | 2,0 | 5 | 5 | 5 | 5 | 5 | 5 | 5 | 5 | 0 |
| 17 | 2,0 | 5 | 5 | 5 | 5 | 5 | 5 | 5 | 5 | 0 |
| 18 | 2,0 | 5 | 5 | 5 | 5 | 5 | 5 | 5 | 5 | 0 |
| 19 | 2,0 | 5 | 5 | 5 | 5 | 5 | 5 | 5 | 5 | 0 |
| 20 | 2,0 | 5 | 5 | 5 | 5 | 5 | 5 | 5 | 5 | 0 |
| 21 | 2,0 | 5 | 5 | 5 | 5 | 5 | 5 | 5 | 5 | 0 |
| 22 | 2,0 | 5 | 5 | 5 | 5 | 5 | 5 | 5 | 5 | 0 |
| 23 | 2,0 | 5 | 5 | 5 | 5 | 5 | 5 | 5 | 5 | 0 |
| 24 | 2,0 | 5 | 5 | 5 | 5 | 5 | 5 | 5 | 5 | 0 |
| 25 | 2,0 | 5 | 5 | 5 | 5 | 5 | 5 | 5 | 5 | 0 |
| 26 | 2,0 | 5 | 5 | 5 | 5 | 5 | 5 | 5 | 5 | 0 |
| 27 | 2,0 | 5 | 5 | 5 | 5 | 5 | 5 | 5 | 5 | 0 |
| 28 | 2,0 | 5 | 5 | 5 | 5 | 5 | 5 | 5 | 5 | 0 |
| 30 | 2,0 | 5 | 5 | 5 | 5 | 5 | 5 | 5 | 5 | 0 |
| 31 | 2,0 | 5 | 5 | 5 | 5 | 5 | 5 | 5 | 5 | 0 |
| 32 | 2,0 | 5 | 5 | 5 | 5 | 5 | 5 | 5 | 5 | 0 |
| 33 | 2,0 | 5 | 5 | 5 | 5 | 5 | 5 | 5 | 5 | 0 |
| 34 | 2,0 | 5 | 5 | 5 | 5 | 5 | 5 | 5 | 5 | 0 |
| 35 | 2,0 | 5 | 5 | 5 | 5 | 5 | 5 | 5 | 5 | 0 |
| 36 | 2,0 | 5 | 5 | 5 | 5 | 5 | 5 | 5 | 5 | 0 |
| 37 | 2,0 | 5 | 5 | 5 | 5 | 5 | 5 | 5 | 5 | 0 |
| 38 | 2,0 | 5 | 5 | 5 | 5 | 5 | 5 | 5 | 5 | 0 |
| 39 | 2,0 | 5 | 5 | 5 | 5 | 5 | 5 | 5 | 5 | 0 |
| 40 | 2,0 | 5 | 5 | 5 | 5 | 5 | 5 | 5 | 5 | 0 |
| 41 | 2,0 | 5 | 5 | 5 | 5 | 5 | 5 | 5 | 5 | 0 |
| 43 | 2,0 | 5 | 5 | 5 | 5 | 5 | 5 | 5 | 5 | 0 |
| 44 | 2,0 | 5 | 5 | 5 | 5 | 5 | 5 | 5 | 5 | 0 |
| 45 | 2,0 | 5 | 5 | 5 | 5 | 5 | 5 | 5 | 5 | 0 |
| 46 | 2,0 | 5 | 5 | 5 | 5 | 5 | 5 | 5 | 5 | 0 |
| 47 | 2,0 | 5 | 5 | 5 | 5 | 5 | 5 | 5 | 5 | 0 |
| 50 | 2,0 | 5 | 5 | 5 | 5 | 5 | 5 | 5 | 5 | 0 |

Tabelle 1 (Fortsetzung).

| Verbindung Nr. | Menge des Wirkstoffs, kg/ha | Herbizide Wirkung Unkräuter | | | | | | | | Phytotoxizität |
|---|---|---|---|---|---|---|---|---|---|---|
| | | A | B | C | D | E | F | G | H | Reis |
| 51 | 2,0 | 5 | 5 | 5 | 5 | 5 | 5 | 5 | 5 | 0 |
| 52 | 2,0 | 5 | 5 | 5 | 5 | 5 | 5 | 5 | 5 | 0 |
| 53 | 2,0 | 5 | 5 | 5 | 5 | 5 | 5 | 5 | 5 | 0 |
| 54 | 2,0 | 5 | 5 | 5 | 5 | 5 | 5 | 5 | 5 | 0 |
| 55 | 2,0 | 5 | 5 | 5 | 5 | 5 | 5 | 5 | 5 | 0 |
| 56 | 2,0 | 5 | 5 | 5 | 5 | 5 | 5 | 5 | 5 | 0 |
| 57 | 2,0 | 5 | 5 | 5 | 5 | 5 | 5 | 5 | 5 | 0 |
| 60 | 2,0 | 5 | 5 | 5 | 5 | 5 | 5 | 5 | 5 | 0 |
| 62 | 2,0 | 5 | 5 | 5 | 5 | 5 | 5 | 5 | 5 | 0 |
| Vergleich(a) | 4,0 | 1 | 0 | 1 | 0 | 1 | 2 | 0 | 2 | 0 |

Bemerkungen

1) Die Nummern der Verbindungen entsprechen den in der nachstehenden Tabelle 2 genannten Verbindungen in den Präparaten.

2) Die Buchstaben A, B, C, D, E, F, G und H in der Spalte mit der Überschrift « Unkräuter » entsprechen den folgenden Unkräutern :

A : Echinochloa crus-galli Beauv. var,
B : Eleocharis acicularis L.,
C : Cyperus iria L.,
D : Scirpus juncoides Roxburgh var,
E : Monochoria vaginalis Presl,
F : Breitblättrige Unkräuter (z. B. Lindernia Probumbens Philcox, Rotala indica Koehne und Elatine triandra Schk),
G : Cyperus serotinus Rottboel und
H : Sagittaria pygmaea Miq.

3) Vergleich (a)

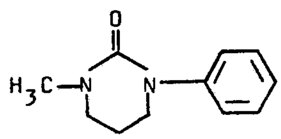

(die in Chemical Abstracts, Vol. 57, 9860a, 1968, beschriebene Verbindung.)

Außerdem wurde als Ergebnis der den vorstehend beschriebenen Versuchen ähnlichen Versuchen bestätigt, daß die Verbindungen Nr. 11, 13, 14, 29, 42, 48, 49, 58, 59 und 61 ausgezeichnete herbizide Aktivität (100 %ige Unkrautbekämpfung bei einer Aufwandmenge der Wirkverbindung von 20 kg/ha) haben, ohne irgendwelche Phytotoxizität gegen Reispflanzen aufzuweisen.

Die Verfahren zur Herstellung der Verbindungen gemäß der Erfindung werden in den folgenden Herstellungsbeispielen ausführlicher beschrieben.

Herstellungsbeispiel 1

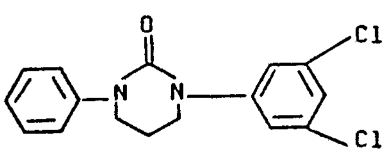

(Verbindung Nr. 1)

15,1 g N-3-Hydroxypropylanilin wurden in 80 ml Chloroform gelöst, und eine Lösung von 18,8 g 3,5-Dichlorophenylisocyanat in 30 ml Chloroform wurde der Lösung bei einer Temperatur von 30 °C oder niedriger zugesetzt. Die Reaktion war leicht exotherm, so daß gekühlt wurde, falls erforderlich. Nachdem einige Zeit bei Raumtemperatur gerührt worden war, wurde der Inhalt 30 Minuten am Rückfluß erhitzt, im

die Harnstoffbildungsreaktion zu vollenden. Dann wurde der Inhalt auf eine Temperatur von 10 °C oder niedriger gekühlt, und nach Zusatz einiger Tropfen Pyridin wurden 14,3 g Thionylchlorid bei einer Temperatur von 10 °C oder niedriger zugesetzt. Das Gemisch wurde einige Zeit bei Raumtemperatur gerührt, und wenn die Entwicklung eines Gases aufhörte, wurde es eine weitere Stunde am Rückfluß erhitzt. Durch Entfernung des überschüssigen Thionylchlorids und Chloroforms unter Druck wurde das rohe Chlorid als Rückstand erhalten. Dieses rohe Chlorid wurde in 50 ml Ethanol gelöst. Der Lösung wurde eine Lösung von 11,2 g Kaliumhydroxid in 40 ml Ethanol zugesetzt und das Gemisch 2 Stunden unter gutem Rühren am Rückfluß erhitzt. Nach der Reaktion wurde der größte Teil des Ethanols unter vermindertem Druck entfernt und der Rückstand in Wasser gegossen, wodurch das gewünschte Produkt in Form von rohen Kristallen ausgefällt wurde. Durch Umkristallisation aus Methanol wurden 27,3 g des gewünschten Produkts, 1-(3,5-Dichlorophenyl)-3-phenyl-tetrahydro-2-pyrimidinon, vom Schmelzpunkt 128 bis 129 °C erhalten.

Herstellungsbeispiel 2

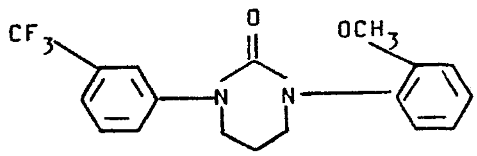

(Verbindung Nr. 2)

23,8 g N-3-Chloropropyl-3-trifluoromethylanilin wurden in 150 ml Toluol gelöst. Der Lösung wurden 14,9 g 2-Methoxyphenylisocyanat zugesetzt, und der Inhalt wurde 3 Stunden unter Rühren bei 50 bis 60 °C erhitzt. Der Inhalt wurde dann auf 30 °C gekühlt, mit einer katalytischen Menge Tetrabutylammoniumbromid versetzt, und unter kräftigem Rühren wurden 25 g einer 50 %igen wäßrigen Natriumhydroxidlösung zugesetzt. Nach erfolgter Zugabe wurde das Gemisch unter Rühren bei 50 °C erhitzt, und nach Abkühlenlassen auf Raumtemperatur wurde die wäßrige Schicht abgetrennt und die Toluolschicht mit Wasser gewaschen. Durch Entfernung des Toluols unter vermindertem Druck wurden 26,3 g des gewünschten Produkts, 1-(2-Methoxyphenyl)-3-(3-trifluoromethyl)-tetrahydro-2-pyrimidinon, als farblose Kristalle vom Schmelzpunkt 97 bis 98°C erhalten.

Herstellungsbeispiel 3

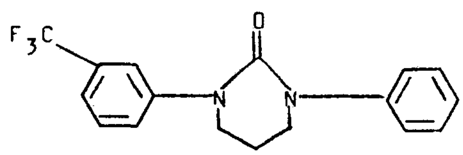

(Verbindung Nr. 3)

29,4 g 1-(3-Trifluoromethylanilino)-3-anilinopropan wurden in 150 ml Toluol gelöst. Der Lösung wurden 22,2 g Triethylamin zugesetzt. Die Lösung wurde auf 0 °C gekühlt, und eine Lösung von 10,9 g Trichloromethylchloroformiat in 20 ml Toluol wurde tropfenweise bei 0° bis 5 °C zugesetzt. Nach erfolgter Zugabe wurde das Gemisch 3 Stunden bei Raumtemperatur gerührt. Der Inhalt wurde mit 1 %iger Salzsäure, einer 1 %igen wäßrigen Natriumhydroxidlösung und Wasser gewaschen und entwässert, worauf das Toluol unter vermindertem Druck entfernt wurde, wobei 25,0 g des gewünschten Produkts, 1-Phenyl-3-(3-trifluoromethylphenyl)-tetrahydro-2-pyrimidinon, vom Schmelzpunkt 101 bis 103 °C erhalten wurden.

In der folgenden Tabelle 2 sind Verbindungen gemäß der Erfindung, die in ähnlicher Weise, wie vorstehend beschrieben, hergestellt wurden, genannt. Hierbei bedeutet Ph = Phenyl ( 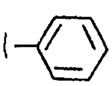 )

Tabelle 2

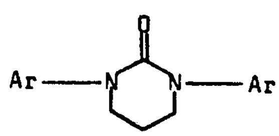

(I)

| Verbindung Nr. | Ar | | Schmelzpunkt, °C |
|---|---|---|---|
| 4 | 3-F-Ph | Ph | 116 - 118.5 |
| 5 | 3-Cl-Ph | Ph | 116 - 117 |
| 6 | 3-Br-Ph | Ph | 117 - 118 |

Tabelle 2 (Fortsetzung)

| Verbindung Nr. | Ar | | Schmelzpunkt, °C |
|---|---|---|---|
| 7 | $3-CH_3-Ph$ | Ph | 78 - 79 |
| 8 | $3-CH_3O-Ph$ | Ph | 110 - 111 |
| 9 | $3-iso-C_3H_7-Ph$ | Ph | 92 - 93.5 |
| 10 | 3-Ph-O-Ph | Ph | 101 - 104 |
| 11 | $3-NO_2-Ph$ | Ph | 131.5 - 132.5 |
| 12 | 3-NC-Ph | Ph | 133 - 135 |
| 13 | $3-CH_3\overset{O}{\overset{\|}{C}}-Ph$ | Ph | 118 - 121 |
| 14 | $3-C_2H_5O\overset{O}{\overset{\|}{C}}-Ph$ | Ph | 111 - 112 |
| 15 | $3-CF_3-Ph$ | 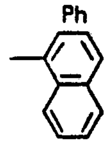 | 118.5 - 120 |
| 16 | 3-F-Ph | $3-CH_3-Ph$ | 115 - 117 |
| 17 | 3-Cl-Ph | 2-Cl-Ph | 122 - 123.5 |
| 18 | 3-Cl-Ph | 3-Cl-Ph | 170.5 - 171.5 |
| 19 | 3-Cl-Ph | 4-Cl-Ph | 162.5 - 163.5 |
| 20 | 3-Cl-Ph | $2-CH_3-Ph$ | 101 - 102 |
| 21 | 3-Cl-Ph | $3-CH_3-Ph$ | 145 - 147.5 |
| 22 | 3-Cl-Ph | $4-CH_3-Ph$ | 167 - 169.5 |
| 23 | 3-Cl-Ph | $2-CH_3O-Ph$ | 117 - 118 |
| 24 | 3-Cl-Ph | $3-CH_3O-Ph$ | 99 - 100 |
| 25 | 3-Cl-Ph | $4-CH_3O-Ph$ | 157 - 158 |
| 26 | 3-Cl-Ph | $3-NO_2-Ph$ | 137 - 138.5 |
| 27 | 3-Cl-Ph | $2-CF_3-Ph$ | 113 - 114 |
| 28 | 3-Cl-Ph | $3-CF_3-Ph$ | 143.5 - 145 |
| 29 | 3-Cl-Ph | $4-CF_3-Ph$ | 125 - 126 |
| 30 | 3-Br-Ph | $3-CF_3-Ph$ | 154 - 155 |
| 31 | $3-CH_3-Ph$ | $2-CH_3-Ph$ | 99.5 - 100.5 |
| 32 | $3-CH_3-Ph$ | $3-CH_3-Ph$ | 117 - 118.5 |
| 33 | $3-CH_3-Ph$ | $4-CH_3-Ph$ | 115.5 - 116.5 |
| 34 | $3-CH_3O-Ph$ | $3-CH_3O-Ph$ | 90 - 93 |
| 35 | $3-CH_3O-Ph$ | $3-CF_3-Ph$ | 123 - 124.5 |
| 36 | $3-NO_2-Ph$ | $2-CH_3-Ph$ | 127 - 130 |
| 37 | 3-NC-Ph | $3-CF_3-Ph$ | 140.5 - 143 |
| 38 | $3-CF_3-Ph$ | 2-Cl-Ph | 118 - 121 |
| 39 | $3-CF_3-Ph$ | 4-Cl-Ph | 126.5 - 127.5 |
| 40 | $3-CF_3-Ph$ | $3-CH_3-Ph$ | 119 - 120 |
| 41 | $3-CF_3-Ph$ | $3-CH_3-Ph$ | 157 - 160 |
| 42 | $3-CF_3-Ph$ | $4-iso-C_3H_7-Ph$ | 133 - 134 |
| 43 | $3-CF_3-Ph$ | $4-CH_3O-Ph$ | 128 - 130 |
| 44 | $3-CF_3-Ph$ | $3-CF_3-Ph$ | 143 - 146 |
| 45 | $2,5-Cl_2-Ph$ | Ph | 127 - 128 |
| 46 | $3,4-Cl_2-Ph$ | Ph | 132 - 133 |
| 47 | $3,5-(CH_3)_2-Ph$ | Ph | 115 - 117 |

Tabelle 2 (Fortsetzung)

| Verbindung Nr. | Ar | | Schmelzpunkt, °C |
|---|---|---|---|
| 48 | 3,4-$(CH_3O)_2$-Ph | Ph | 120 - 122 |
| 49 | 3-Cl,4-$CF_3$-Ph | Ph | 139 - 140.5 |
| 50 | 3,5-$(CF_3)_2$-Ph | Ph | 176 - 178 |
| 51 | 3-Cl-Ph | 3,5-$Cl_2$-Ph | 117 - 118 |
| 52 | 3-$CF_3$-Ph | 3,5-$Cl_2$-Ph | 94 - 95.5 |
| 53 | 3-$CF_3$-Ph | 2,6-$(CH_3)_2$-Ph | 113 - 114 |
| 54 | 3-$CF_3$-Ph | 3,4-$(CH_3)_2$-Ph | 165 - 166.5 |
| 55 | 3-$CF_3$-Ph | 3-Cl,4-$CF_3$-Ph | 124 - 125 |
| 56 | 3,4-$Cl_2$-Ph | 2-$CH_3$-Ph | 146 - 147.5 |
| 57 | 2,4,5-$Cl_3$-Ph | Ph | 174 - 175 |
| 58 | 3-$CF_3$-Ph | 2,4,5-$Cl_3$-Ph | 147 - 150 |
| 59 | 3,5-$(CH_3O)_2$-Ph | Ph | 95.5 - 96.5 |
| 60 | 3-Ph-O-Ph | 3-$CF_3$-Ph | 101 - 102 |
| 61 | 3,5-$Cl_2$-Ph | 2-$CH_3O$-Ph | 160 - 161 |
| 62 | 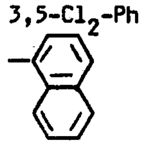 | Ph | 177 - 178 |

**Ansprüche** (für die Vertragsstaaten : BE, CH, DE, FR, GB, IT, LI, NL, SE)

1. Substituiertes Tetrahydropyrimidinonderivat der allgemeinen Formel

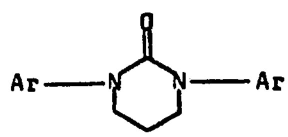

(I)

worin die Gruppen Ar gleich oder verschieden sein können und jeweils aus einer α-Naphthylgruppe und einer Gruppe der Formel

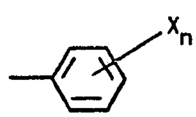

ausgewählt sind, worin X ein Rest ist, der aus der aus einem Halogenatom, einem Alkylrest mit 1-4 C-Atomen, einem Alkoxyrest mit 1-4 C-Atomen, einer Nitrogruppe, einer Cyanogruppe, einer Alkylcarbonylgruppe mit 1-4 C-Atomen im Alkylteil, einer Alkoxycarbonylgruppe mit 1-4 C-Atomen im Alkoxyteil, einem Trifluormethylrest und einer Phenoxygruppe bestehenden Gruppe ausgewählt ist, wobei in Fällen in denen mehrere Gruppen X vorhanden sind, diese gleich oder verschieden sein können, und n für 0, 1, 2 oder 3 steht.

2. 1-(2-Methoxyphenyl)-3-(trifluoromethylphenyl)-tetrahydro-2-pyrimidinon nach Anspruch 1 mit der Formel

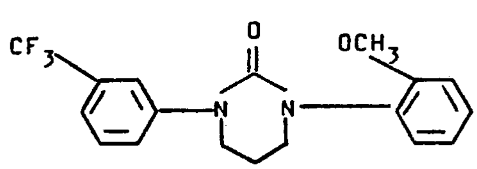

3. 1-(3,5-Dichlorophenyl)-3-phenyl-tetrahydro-2-pyrimidinon nach Anspruch 1 mit der Formel

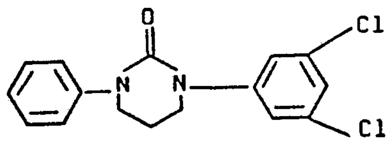

4. Verfahren zur Herstellung von substituierten Tetrahydropyrimidinonderivaten der allgemeinen Formel (I) gemäß Anspruch 1, dadurch gekennzeichnet, daß man eine Verbindung der allgemeinen Formel

$$Ar—NH(CH_2)_3OH \quad (II)$$

in der Ar die vorstehend genannte Bedeutung hat, und ein Isocyanat der allgemeinen Formel

$$Ar—N = C = O \quad (III)$$

in der Ar die vorstehend genannte Bedeutung hat und die gleiche Gruppe Ar oder verschiedenen von der anderen Gruppe Ar sein kann, und anschließend mit einem Halogenierungsmittel und dann mit einem Alkalihydroxid umsetzt.

5. Verfahren zur Herstellung von substituierten Tetrahydropyrimidinonderivaten der allgemeinen Formel (I) gemäß Anspruch 1, dadurch gekennzeichnet, daß man eine Verbindung der allgemeinen Formel

$$Ar—NH(CH_2)_3Y \quad (IV)$$

in der Ar die vorstehend genannte Bedeutung hat und Y ein Halogenatom ist, und ein Isocyanat der allgemeinen Formel

$$Ar—N = C = O \quad (III)$$

in der Ar die vorstehend genannte Bedeutung hat und die gleiche Gruppe Ar oder verschieden von der anderen Gruppe Ar sein kann, und anschließend mit einem Alkalihydroxid umsetzt.

6. Verfahren zur Herstellung von substituierten Tetrahydropyrimidinonderivaten der allgemeinen Formel (I) gemäß Anspruch 1, dadurch gekennzeichnet, daß man eine Verbindung der allgemeinen Formel

$$Ar—NH(CH_2)_3NH—Ar \quad (V)$$

in der die Gruppen Ar die vorstehend genannte Bedeutung haben, und Phosgen oder Trichloromethylchloroformiat umsetzt.

7. Herbizide Mittel, gekennzeichnet durch einen Gehalt an mindestens einem substituierten Tetrahydropyrimidinonderivat der Formel (I) gemäß Anspruch 1.

8. Verwendung von substituierten Tetrahydropyrimidinonderivaten der Formel (I) gemäß Anspruch 1 Bekämpfung von Unkraut.

9. Verfahren zur Herstellung von herbiziden Mitteln, dadurch gekennzeichnet, daß man substituierte Tetrahydropyrimidinonderivate der Formel (I) gemäß Anspruch 1 mit Streckmitteln und/oder oberflächenaktiven Mitteln vermischt.

**Ansprüche** (für den Vertragsstaat, AT)

1. Verfahren zur Herstellung eines substituierten Tetrahydropyrimidinonderivats der allgemeinen Formel

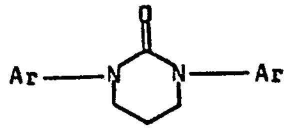

(I)

worin die Gruppen Ar gleich oder verschieden sein können und jeweils aus einer α-Naphthylgruppe und einer Gruppe der Formel

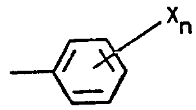

ausgewählt sind, worin X ein Rest ist, der aus der aus einem Halogenatom, einem Alkylrest mit 1-4 C-Atomen, einem Alkoxyrest mit 1-4 C-Atomen, einer Nitrogruppe, einer Cyanogruppe, einer Alkylcarbonylgruppe mit 1-4 C-Atomen im Alkylteil, einer Alkoxycarbonylgruppe mit 1-4 C-Atomen im Alkoxyteil, einem Trifluormethylrest und einer Phenoxygruppe bestehenden Gruppe ausgewählt ist, wobei in Fällen, in denen mehrere Gruppen X vorhanden sind, diese gleich oder verschieden sein können, und n für 0, 1, 2 oder 3 steht, dadurch gekennzeichnet, daß man eine Verbindung der allgemeinen Formel

$$Ar—NH(CH_2)_3OH \qquad (II)$$

in der Ar die vorstehend genannte Bedeutung hat, und ein Isocyanat der allgemeinen Formel

$$Ar—N = C = O \qquad (III)$$

in der Ar die vorstehend genannte Bedeutung hat und die gleiche Gruppe Ar oder verschiedenen von der anderen Gruppe Ar sein kann, und anschließend mit einem Halogenierungsmittel und dann mit einem Alkalihydroxid umsetzt.

2. Verfahren zur Herstellung von substituierten Tetrahydropyrimidinonderivaten der allgemeinen Formel (I) gemäß Anspruch 1, dadurch gekennzeichnet, daß man eine Verbindung der allgemeinen Formel

$$Ar—NH(CH_2)_3Y \qquad (IV)$$

in der Ar die vorstehend genannte Bedeutung hat und Y ein Halogenatom ist, und ein Isocyanat der allgemeinen Formel

$$Ar—N = C = O \qquad (III)$$

in der Ar die vorstehend genannte Bedeutung hat und die gleiche Gruppe Ar oder verschieden von der anderen Gruppe Ar sein kann, und anschließend mit einem Alkalihydroxid umsetzt.

3. Verfahren zur Herstellung von substituierten Tetrahydropyrimidinonderivaten der allgemeinen Formel (I). gemäß Anspruch 1, dadurch gekennzeichnet, daß man eine Verbindung der allgemeinen Formel

$$Ar—NH(CH_2)_3NH—Ar \qquad (V)$$

in der die Gruppen Ar die vorstehend genannte Bedeutung haben, und Phosgen oder Trichloromethylchloroformiat umsetzt.

4. Herbizide Mittel, gekennzeichnet durch einen Gehalt an mindestens einem substituierten Tetrahydropyrimidinonderivat der Formel (I) gemäß Anspruch 1.

5. Verwendung von substituierten Tetrahydropyrimidinonderivaten der Formel (I) gemäß Anspruch 1 zur Bekämpfung von Unkraut.

6. Verfahren zur Herstellung von herbiziden Mitteln, dadurch gekennzeichnet, daß man substituierte Tetrahydropyrimidinonderivate der Formel (I) gemäß Anspruch 1 mit Streckmitteln und/oder oberflächenaktiven Mitteln vermischt.

**Claims** (for the Contracting : BE, CH, DE, FR, GB, IT, LI, NL, SE)

1. Substituted tetrahydropyrimidinone derivative of the general formula

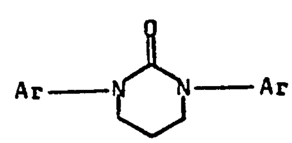

(I)

wherein the groups Ar can be identical or different and are each selected from an α-naphthyl group or a group of the formula

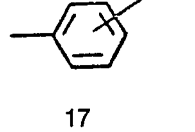

wherein X is a radical which is selected from the group consisting of a halogen atom, an alkyl radical with 1-4 C-atoms, an alkoxy radical with 1-4 C-atoms, a nitro group, a cyano group, an alkylcarbonyl group with 1-4 C-atoms in the alkyl part, an alkoxycarbonyl group with 1-4 C-atoms in the alkoxy part, a trifluoromethyl radical and a phenoxy group, wherein in cases where more than one groupe X are present these can be identical or different, and n represents 0, 1, 2 or 3.

2. 1-(2-Methoxyphenyl)-3-(trifluoromethylphenyl)-tetrahydro-2-pyrimidinone according to Claim 1, having the formula

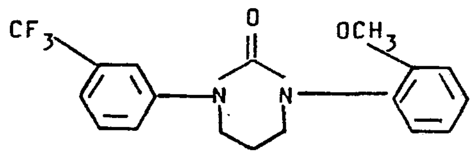

3. 1-(3,5-Dichlorophenyl)-3-phenyl-tetrahydro-2-pyrimidinone according to Claim 1, having the formula

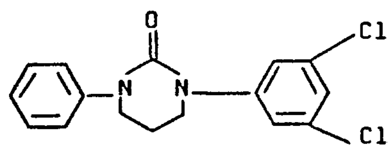

4. Process for the preparation of substituted tetrahydropyrimidinone derivatives of the general formula (I) according to Claim 1, characterised in that a compound of the general formula

$$Ar—NH(CH_2)_3OH \qquad (II)$$

in which Ar has the above-mentioned meaning, and an isocyanate of the general formula

$$Ar—N=C=O \qquad (III)$$

in which Ar has the above-mentioned meaning and can be the same group Ar or different from the other group Ar, are reacted and the product is then reacted with a halogenating agent and then with an alkali metal hydroxide.

5. Process for the preparation of substituted tetrahydropyrimidinone derivatives of the general formula (I) according to Claim 1, characterised in that a compound of the general formula

$$Ar—NH(CH_2)_3Y \qquad (IV)$$

in which Ar has the above-mentioned meaning and Y is a halogen atom, and an isocyanate of the general formula

$$Ar—N=C=O \qquad (III)$$

in which Ar has the above-mentioned meaning and can be the same group Ar or different from the other group Ar, are reacted and the product is then reacted with an alkali metal hydroxide.

6. Process for the preparation of substituted tetrahydropyrimidinone derivatives of the general formula (I) according to Claim 1, characterised in that a compound of the general formula

$$Ar—NH(CH_2)_3NH—Ar \qquad (V)$$

in which the groups Ar have the above-mentioned meaning, and phosgene or trichloromethyl chloroformate are reacted.

7. Herbicidal agents, characterised in that they contain at least one substituted tetrahydropyrimidinone derivative of the formula (I) according to Claim 1.

8. Use of substituted tetrahydropyrimidinone derivatives of the formula (I) according to Claim 1 for combating weeds.

9. Process for the preparation of herbicidal agents, characterised in that substituted tetrahydropyrimidinone derivatives of the formula (I) according to Claim 1 are mixed with extenders and/or surface-active agents.

**Claims** (for the Contracting State, AT)

1. Process for the preparation of a substituted tetrahydropyrimidinone derivative of the general formula

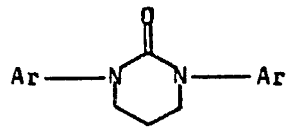

(I)

wherein the groups Ar can be identical or different and are each selected from an α-naphthyl group or a group of the formula

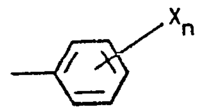

wherein X is a radical which is selected from the group consisting of a halogen atom, an alkyl radical with 1-4 C-atoms, an alkoxy radical with 1-4 C-atoms, an alkoxy radical with 1-4 C-atoms, a nitro group, a cyano group, an alkylcarbonyl group with 1-4 C-atoms in the alkyl part, an alkoxycarbonyl group with 1-4 C-atoms in the alkoxy part, a trifluoromethyl radical and a phenoxy group, wherein in cases where more than one group X are present these can be identical or different, and n represents 0, 1, 2 or 3, characterised in that a compound of the general formula

$$Ar—NH(CH_2)_3OH \qquad (II)$$

in which Ar has the above-mentioned meaning, and an isocyanate of the general formula

$$Ar—N = C = O \qquad (III)$$

in which Ar has the above-mentioned meaning and can be the same group Ar or different from the other group Ar, are reacted and the product is then reacted with a halogenating agent and then with an alkali metal hydroxide.

2. Process for the preparation of substituted tetrahydropyrimidinone derivatives of the general formula (I) according to Claim 1, characterised in that a compound of the general formula

$$Ar—NH(CH_2)_3Y \qquad (IV)$$

in which Ar has the above-mentioned meaning and Y is a halogen atom, and an isocyanate of the general formula

$$Ar—N = C = 0 \qquad (III)$$

in which Ar has the above-mentioned meaning and can be the same group Ar or different from the other group Ar, are reacted and the product is then reacted with an alkali metal hydroxide.

3. Process for the preparation of substituted tetrahydropyrimidinone derivatives of the general formula (I) according to Claim 1, characterised in that a compound of the general formula

$$Ar—NH(CH_2)_3NH—Ar \qquad (V)$$

in which the groups Ar have the above-mentioned meaning, and phosgene or trichloromethyl chloroformate are reacted.

4. Herbicidal agents, characterised in that they contain at least one substituted tetrahydropyrimidinone derivative of the formula (I) according to Claim 1.

5. Use of substituted tetrahydropyrimidinone derivatives of the formula (I) according to Claim 1 for combating weeds.

6. Process for the preparation of herbicidal agents, characterised in that substituted tetrahydropyrimidinone derivatives of the formula (I) according to Claim 1 are mixed with extenders and/or surface-active agents.

**Revendications** (pour les Etats contractants: BE, CH, DE, FR, GB, IT, LI, NL, SE)

1. Dérivé substitué de tétrahydropyrimidinone de formule générale

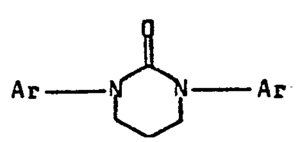

(I)

dans laquelle les groupes Ar peuvent être égaux ou différents et sont choisis chacun entre un groupe α-naphtyle et un groupe de formule

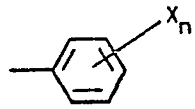

où X est un reste qui est choisi dans le groupe formé d'un atome d'halogène, d'un reste alkyle ayant 1 à 4 atomes de carbone, d'un reste alkoxy ayant 1 à 4 atomes de carbone, d'un groupe nitro, d'un groupe cyano, d'un groupe alkylcarbonyle ayant 1 à 4 atomes de carbone dans la partie alkyle, d'un groupe alkoxycarbonyle ayant 1 à 4 atomes de carbone dans la partie alkoxy, d'un reste trifluorométhyle et d'un groupe phénoxy, et dans les cas où il existe plusieurs groupes X, ces groupes peuvent être égaux ou différents, et n est égal à 0, 1, 2 ou 3.

2. La 1-(2-méthoxyphényl)-3-(trifluorométhylphényl)tétrahydro-2-pyrimidinone suivant la revendication 1, de formule

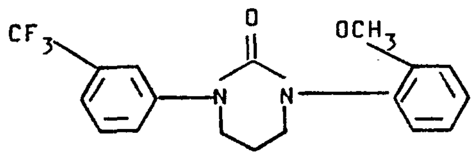

3. La 1-(3,5-dichlorophényl)-3-phényltétrahydro-2-pyrimidinone suivant la revendication 1, de formule

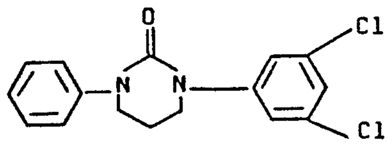

4. Procédé de production de dérivés substitués de tétrahydropyrimidinone de formule générale (I) suivant la revendication 1, caractérisé en ce qu'on fait réagir un composé de formule générale

$$Ar—NH(CH_2)_3OH \quad (II)$$

dans laquelle Ar a la définition mentionnée ci-dessus, et un isocyanate de formule générale

$$Ar—N=C=O \quad (III)$$

dans laquelle Ar a la définition mentionnée ci-dessus et peut être le même que l'autre groupe Ar ou peut en être différent, puis on le fait réagir avec un agent d'halogénation et, ensuite, avec un hydroxyde alcalin.

5. Procédé de production de dérivés substitués de tétrahydropyrimidinone de formule générale (I) suivant la revendication 1, caractérisé en ce qu'on fait réagir un composé de formule générale

$$Ar—NH(CH_2)_3Y \quad (IV)$$

dans laquelle Ar a la définition mentionnée ci-dessus et Y est un atome d'halogène, et un isocyanate de formule générale

$$Ar—N=C=O \quad (III)$$

dans laquelle Ar a la définition mentionnée ci-dessus et peut être le même que l'autre groupe Ar ou peut en être différent, et on fait ensuite réagir avec un hydroxyde alcalin.

6. Procédé de production de dérivés substitués de tétrahydropyrimidinone de formule générale (I) suivant la revendication 1, caractérisé en ce qu'on fait réagir un composé de formule générale

$$Ar—NH(CH_2)_3NH—Ar \quad (V)$$

dans laquelle les groupes Ar ont la définition indiquée ci-dessus, et du phosgène ou du chloroformiate de trichlorométhyle.

7. Compositions herbicides, caractérisées par une teneur en au moins un dérivé substitué de tétrahydropyrimidinone de formule (I) suivant la revendication 1.

8. Utilisation de dérivés substitués de tétrahydropyrimidinone de formule (I) suivant la revendication 1 pour combattre des mauvaises herbes.

9. Procédé de production de compositions herbicides, caractérisé en ce qu'on mélange des dérivés substitués de tétrahydropyrimidinone de formule (I) suivant la revendication 1 avec des diluants et/ou des agents tensioactifs.

**Revendications** (pour l'Etat contractant, AT)

1. Procédé de production d'un dérivé substitué de tétrahydropyrimidinone de formule générale

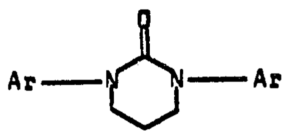

(I)

dans laquelle les groupes Ar peuvent être égaux ou différents et sont choisis chacun entre un groupe α-naphtyle et un groupe de formule

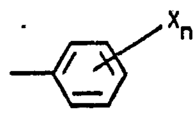

où X est un reste qui est choisi entre un atome d'halogène, un reste alkyle ayant 1 à 4 atomes de carbone, un reste alkoxy ayant 1 à 4 atomes de carbone, un groupe nitro, un groupe cyano, un groupe alkylcarbonyle ayant 1 à 4 atomes de carbone dans la partie alkyle, un groupe alkoxycarbonyle ayant 1 à 4 atomes de carbone dans la partie alkoxy, un reste trifluorométhyle et un groupe phénoxy, et, dans les cas où plusieurs groupes X sont présents, ces groupes peuvent être égaux ou différents, et n est égal à 0, 1, 2, 3 ou 4, caractérisé en ce qu'on fait réagir un composé de formule générale

$$Ar—NH(CH_2)_3OH \qquad (II)$$

dans laquelle Ar a la définition mentionnée ci-dessus, et un isocyanate de formule générale

$$Ar—N=C=O \qquad (III)$$

dans laquelle Ar a la définition indiquée ci-dessus et peut être le même que l'autre groupe Ar ou peut en être différent, puis on fait réagir avec un agent d'halogénation et ensuite avec un hydroxyde alcalin.

2. Procédé de production de dérivés substitués de tétrahydropyrimidinone de formule générale (I) suivant la revendication 1, caractérisé en ce qu'on fait réagir un composé de formule générale

$$Ar—NH(CH_2)_3Y \qquad (IV)$$

dans laquelle Ar a la définition mentionnée ci-dessus et Y est un atome d'halogène, et un isocyanate de formule générale

$$Ar—N=C=O \qquad (III)$$

dans laquelle Ar a la définition mentionnée ci-dessus et peut être le même que l'autre groupe Ar ou peut en être différent, puis on fait réagir avec un hydroxyde alcalin.

3. Procédé de production de dérivés substitués de tétrahydropyrimidinone de formule générale (I) suivant la revendication 1, caractérisé en ce qu'on fait réagir un composé de formule générale

$$Ar—NH(CH_2)_3NH—Ar \qquad (V)$$

dans laquelle les groupes Ar ont la définition mentionnée ci-dessus, et du phosgène ou du chloroformiate de trichlorométhyle.

4. Compositions herbicides, caractérisées par une teneur en au moins un dérivé substitué de tétrahydropyrimidinone de formule (I) suivant la revendication 1.

5. Utilisation de dérivés substitués de tétrahydropyrimidinone de formule (I) suivant la revendication 1 pour combattre des mauvaises herbes.

6. Procédé de production de compositions herbicides, caractérisé en ce qu'on mélange des dérivés substitués de tétrahydropyrimidinone de formule (I) suivant la revendication 1 avec des diluants et/ou des agents tensioactifs.